# EUROPÄISCHE PATENTSCHRIFT

(11) **EP 2 857 063 B1**
(45) Veröffentlichungstag und Bekanntmachung des Hinweises auf die Patenterteilung: **12.10.2016**
(21) Anmeldenummer: 14152769.7
(22) Anmeldetag: 28.01.2014
(51) Int. Cl.: A61N 1/04, A61N 1/36, A61F 7/00

(54) **Vorrichtung zur Elektrostimulation sowie Verfahren zur Ansteuerung von mehreren Elektroden in einer Vorrichtung zur Elektrostimulation**
Device for electrostimulation, and method for controlling multiple electrodes in a device for electrostimulation
Dispositif d'électrostimulation et procédé de commande de plusieurs électrodes dans un dispositif d'électrostimulation

(30) Priorität: 02.10.2013 DE 102013110984
(43) Veröffentlichungstag der Anmeldung: 08.04.2015
(73) Patentinhaber: Pierenkemper GmbH, 35630 Ehringshausen (DE)
(72) Erfinder: Pierenkemper, Roger, D-35037 Marburg (DE)
(74) Vertreter: Knefel, Cordula

(56) Entgegenhaltungen:
- US-A1- 2003 114 892
- US-A1- 2010 004 715
- US-A1- 2010 087 903
- US-A1- 2010 152 794
- US-A1- 2011 015 697
- US-A1- 2011 093 030
- US-A1- 2011 264 002
- US-A1- 2012 041 513
- US-A1- 2013 085 317
- US-B2- 8 145 318

## Beschreibung

Die Erfindung betrifft eine Vorrichtung zur Elektrostimulation mit einer Teilkörper- oder Ganzkörperauflage und in der Auflage angeordneten Elektroden. Darüber hinaus betrifft die Erfindung ein Verfahren zur Ansteuerung von mehreren Elektroden in einer Vorrichtung zur Elektrostimulation.

Aus der Praxis sind lokal anwendbare Vorrichtungen zur Elektrostimulation, beispielsweise Arm- oder Beinmanschetten bekannt.

Aus der koreanischen Druckschrift KR 10-2012-00041525 sind Matten bekannt, die als Vorrichtung zur Elektrostimulation ausgebildet sind. Die Ganzkörpermatten gemäß diesem Stand der Technik weisen Elektroden auf, die im Bereich des Körpers einer zu behandelnden Person angeordnet sind. Die Elektroden sind linien- oder punktförmig ausgebildet, wodurch der Nachteil auftritt, dass die Elektrostimulation der Nerven oder Muskeln sehr eingeschränkt ist. Eine effektive Nerven- oder Muskelstimulation ist mit diesen punkt- oder linienförmig ausgestalteten Elektroden, die in einer plan ausgebildeten Matte angeordnet sind, nicht möglich.

Mit einer Elektrostimulation können Muskeln und/oder Nerven stimuliert werden.

Bei einer Nervenstimulation wird eine elektrische Feldstärke mit genügend starkem Gradienten angelegt, die im Nerv die Auslösung eines Aktionspotentials bewirkt, welches entlang des Motornerves zur motorischen Endplatte des adressierten Muskels gelangt, dort erneut ein Aktionspotential auslöst, welches in weiterer Folge eine Kontraktion des Muskels bewirkt. Neben motorischen Nerven werden auch oder ausschließlich sensible Nerven erregt.

Bei einer direkten Muskelzellstimulation wird eine Muskelzelle direkt durch elektrische Reize erregt, die bedeutend größer und länger sind als bei der Stimulation von Nerven. In beiden Fällen ist es bekannt, diese Elektrostimulation mit Oberflächenelektroden über die Haut durchzuführen.

Häufig wird bei einer Muskelstimulation mittelfrequenter Strom eingesetzt, der mit Frequenzen von beispielsweise über 1.000 Hertz (Hz) arbeitet, so dass die sensible Belastung deutlich herabgesetzt wird, da der elektrische Widerstand der Haut gegenüber einem elektrischen Reiz umgekehrt proportional frequenzabhängig ist. Als besonders effektiv haben sich hier Frequenzen zwischen 1.000 und 10.000 Hz und modulierte Ströme herausgestellt.

Aus dem Stand der Technik (US 2011/0093030 A1) ist eine Programmiertechnik für eine medizinische Vorrichtung offenbart. Diese medizinische Vorrichtung ist in der Art eines Hirnschrittmachers aufgebaut. Gemäß diesem Stand der Technik werden Nerven stimuliert, um Hirnareale, beispielsweise von parkinsonerkrankten Personen zu stimulieren. Eine effektive Muskelstimulation ist dieser Druckschrift nicht zu entnehmen.

Weiterhin gehört zum Stand der Technik (US 2010/0152794 A1) eine Vorrichtung, die lediglich einzelne Elektroden ansteuert, um Nervenrezeptoren, beispielsweise in Fingerspitzen zu aktivieren. Eine intensive Muskelstimulation ist mit dieser zum Stand der Technik gehörenden Vorrichtung nicht möglich.

Zum Stand der Technik (US 2010/0087903 A1) gehört eine Vorrichtung zur Messung des Widerstandes auf der Haut, um eine optimale Position für eine Elektrode zu ermitteln. Eine optimierte Muskelstimulation ist mit dieser Vorrichtung nicht möglich.

Weiterhin gehört zum Stand der Technik (US 2011/0015697 A1) eine Vorrichtung, mit der eine Bewertung einer Wundheilung durchgeführt wird. Eine optimierte Muskelstimulation ist mit dieser Vorrichtung ebenfalls nicht möglich.

Aus dem Stand der Technik (US 2011/0264002 A1) ist darüber hinaus eine Vorrichtung bekannt, mit der ebenfalls gemessen wird, ob eine Elektrode korrekt sitzt. Mit dieser Vorrichtung wird eine Nervenstimulation einer Hand durchgeführt, um eine Muskelreaktion zu erzielen. Eine optimierte Muskelstimulation ist mit dieser Vorrichtung ebenfalls nicht möglich.

Zum Stand der Technik (US 8,145,318 B2) gehört darüber hinaus eine Vorrichtung zur Bestimmung einer optimalen Elektrodenposition. Eine effektive Stimulation von Muskeln ist mit dieser Vorrichtung ebenfalls nicht möglich.

Das der Erfindung zugrunde liegende technische Problem besteht darin, eine Vorrichtung und ein Verfahren zur Elektrostimulation anzugeben, mit denen eine deutlich verbesserte Elektrostimulation möglich ist.

Dieses technische Problem wird durch eine Vorrichtung mit den Merkmalen gemäß Anspruch 1 sowie durch ein Verfahren mit den Merkmalen gemäß Anspruch 14 gelöst.

Die erfindungsgemäße Vorrichtung zur Elektrostimulation mit einer Teilkörper- oder Ganzkörperauflage und in der Auflage angeordneten Elektroden, bei der die Elektroden paarweise zu einer Mittellängsachse der Vorrichtung angeordnet sind, wobei die Elektroden flächig ausgebildet sind, und wobei Elektrodenpaare, die aus benachbarten Elektroden gebildet sind, nacheinander oder gleichzeitig ansteuerbar ausgebildet sind, zeichnet sich dadurch aus, dass wenigstens ein Teil der Elektroden eine gekrümmte Oberfläche aufweist, und/oder dass wenigstens ein Teil der Elektroden zu einer Grundfläche der Auflage geneigt angeordnet ist, und dass die gekrümmte Oberfläche der Elektroden als eine an eine Körperkontur angepasste, gekrümmte Oberfläche ausgebildet ist und/oder dass die Neigung der Elektroden als eine an eine Körperkontur angepasste Neigung ausgebildet ist, dass die Vorrichtung eine einer Körperkontur angepasste Liegefläche aufweist, dass die Liegefläche in einer Mittellängsachse eine durchgehende Ausnehmung aufweist, dass die Vorrichtung eine unterbrochene Liegefläche aufweist, und dass Auflageflächen beweglich ausgebildet sind, derart, dass die Elektroden sich optimal einer Körperkontur einer auf der Vorrichtung liegenden Person anpassend ausgebildet sind.

Bevorzugt weist die erfindungsgemäße Vorrichtung eine Rückenauflage auf oder ist als Auflagefläche im Bereich eines Rückens ausgebildet. Es ist jedoch auch möglich, die erfindungsgemäße Vorrichtung für andere Bereiche des Körpers auszubilden, beispielsweise für den Arm- oder Beinbereich.

Die Erfindung weist den Vorteil auf, dass die Elektroden paarweise zu der Mittellängsachse angeordnet sind. Hierdurch ist eine Ganzkörperstimulation vom Kopf bis zu den Füßen oder in dem Teilbereich des Körpers, der durch die erfindungsgemäße Vorrichtung abgedeckt ist, beispielsweise des Rückens möglich.

Durch die Anordnung paarweise zu einer Mittellängsächse bei einer Vorrichtung, die als Rückenauflage oder Ganzkörperauflage ausgebildet ist, werden beide Körperhälften rechts und links der Wirbelsäule stimuliert.

Die Stimulation erfolgt über benachbarte Elektrodenpaare, damit ein Stromfluss zwischen jeweils zwei benachbarten Elektroden vorhanden ist. Die benachbarten Elektrodenpaare können rechts und links von der Wirbelsäule bei Ausbildung der Vorrichtung als Rückenauflage ausgewählt werden. Es besteht auch die Möglichkeit, benachbarte Elektrodenpaare lediglich links oder rechts von der Wirbelsäule mit Strom zu beaufschlagen. Die Elektrodenpaare, die aus benachbarten Elektroden gebildet werden, können nacheinander oder gleichzeitig angesteuert werden.

Die erfindungsgemäße Vorrichtung zeichnet sich darüber hinaus dadurch aus, dass die Elektrodenpaare gleichzeitig oder nacheinander angesteuert werden können. Hierdurch ist es möglich, beispielsweise eine wellenförmige Ansteuerung der Elektrodenpaare und damit eine wellenförmige Stimulation des Körpers vorzusehen.

Vorteilhaft sind je zwei Elektroden paarweise zu der Mittellängsachse der Vorrichtung angeordnet. Da die Elektroden flächig ausgebildet sind, weisen sie eine Größe auf, die den zu stimulierenden Bereich großflächig oder sogar vollständig abdeckt. Hierbei ist es ausreichend, zu beiden Seiten der Mittellängsachse jeweils eine Elektrode vorzusehen. Hierdurch verringert sich der Verkabelungsaufwand in der Vorrichtung und der Aufwand hinsichtlich der Ansteuerung der Elektroden erheblich. Ein wesentlicher Vorteil der flächigen Ausbildung der Elektroden ist auch, dass die Elektroden die zu stimulierenden Muskelbereiche mit einer größeren Fläche berühren und hierdurch eine effektivere Muskelstimulation möglich ist.

Gemäß der Erfindung weist wenigstens ein Teil der Elektroden eine gekrümmte Oberfläche auf. Dadurch, dass die Elektroden flächig ausgebildet sind, ist die Ausbildung mit einer gekrümmten Oberfläche vorteilhaft, da in diesem Fall die Elektroden der Körperkontur angepasst ausgebildet werden können. Beispielsweise können die Elektroden im Halswirbelbereich konvex ausgebildet sein, während sie im Lendenwirbelbereich vorteilhaft konkav ausgebildet sind.

Gemäß der Erfindung ist wenigstens ein Teil der Elektroden zu einer Grundfläche der Auflage geneigt angeordnet. Auch hierdurch ist eine optimale Anpassung der Elektroden an eine Körperkontur möglich.

Die Vorrichtung ist derart ausgebildet, dass die gekrümmte Oberfläche der Elektroden als eine an eine Körperkontur angepasste gekrümmte Oberfläche ausgebildet ist und/oder dass die Neigung der Elektroden als eine an eine Körperkontur angepasste Neigung ausgebildet ist. Hierdurch wird erreicht, dass die Elektroden an dem Körper anliegen und dadurch eine optimale Elektrostimulation möglich ist.

Vorteilhaft sind wenigstens sechs Elektrodenpaare, besonders vorteilhaft wenigstens zwölf Elektrodenpaare vorgesehen.

Durch die Vielzahl von Elektrodenpaaren ist die wellenförmige Stimulation besonders gut möglich, das bedeutet, dass beispielsweise im Kreuzbeinbereich mit der Stimulation angefangen werden kann und dass sich die Stimulation bis zum Halswirbelsäulenbereich durch Ansteuerung der Elektrodenpaare nacheinander fortsetzen kann, wenn die Matte als Rückenauflage ausgebildet ist.

Gemäß der Erfindung ist vorteilhaft vorgesehen, dass die Elektrodenpaare jeweils anschwellend und wieder abschwellend ansteuerbar ausgebildet sind. Das bedeutet, dass vorteilhaft ein erstes Elektrodenpaar anschwellend und wieder abschwellend angesteuert wird. Vorteilhaft wird während des Abschwellvorganges des ersten Elektrodenpaares das nächste folgende Elektrodenpaar angesteuert, so dass dieses anschwillt, so dass eine wellenförmige Elektrostimulation mit der erfindungsgemäßen Vorrichtung möglich ist.

Es ist möglich, die Vorrichtung mit lediglich einer Stimulationswelle durchlaufen zu lassen, das heißt, dass ein erstes Elektrodenpaar anschwellend und wieder abschwellend angesteuert wird. Während des Abschwellvorganges des ersten Elektrodenpaares wird das nächstfolgende benachbarte Elektrodenpaar anschwellend ausgebildet. Während dieser Zeit wird kein anderes Elektrodenpaar angesteuert.

Es besteht auch die Möglichkeit, die Elektrostimulation in mehreren Wellen gleichzeitig durchzuführen. Hierbei wird ein erstes Elektrodenpaar und wenigstens ein weiteres, nicht benachbartes Elektrodenpaar anschwellend und anschließend wieder abschwellend angesteuert. Nachfolgend wird das jeweils benachbarte, nachfolgende Elektrodenpaar anschwellend und wieder abschwellend angesteuert, so dass eine wellenförmige Elektrostimulation entsteht.

Vorteilhaft wird die Stimulation mit einem Strom mit einer Niederfrequenz zwischen 0 und 1.000 Hertz (Hz) oder einer Mittelfrequenz zwischen ein und zehn Kilohertz (kHz) durchgeführt. Diese Frequenzbereiche sind für eine Elektrostimulation im muskulären Bereich besonders vorteilhaft.

Gemäß einer weiteren vorteilhaften Ausführungsform der Erfindung ist die Vorrichtung erwärmbar ausgebildet. Dies erhöht den Stimulationseffekt, da sich die Muskeln hierdurch bei der Stimulation gut und/oder schneller entspannen.

Gemäß einer weiteren vorteilhaften Ausführungsform der Erfindung weist die Vorrichtung eine einer Körperkontur angepasste Liegefläche auf. Auch hierdurch entspannen sich die Muskeln besonders gut und besonders schnell, so dass die Elektrostimulation besonders effektiv ist.

Gemäß einer weiteren vorteilhaften Ausführungsform der Erfindung ist die Vorrichtung aus Silikon gebildet. Die Verwendung von Silikon weist den Vorteil auf, dass zum einen die Elektroden sehr gut in dem Silikon eingebettet werden können. Darüber hinaus lässt sich dieses Material leicht sauber halten, so dass die erfindungsgemäße Vorrichtung auch speziellen Hygienevorschriften entspricht.

Das Silikon und die hieraus gebildeten Auflagen sind weich und nachgiebig, wodurch wiederum der Entspannungseffekt deutlich besser und schneller eintritt und hierdurch der Wirkungsgrad der Elektrostimulation erhöht wird.

Gemäß einer weiteren vorteilhaften Ausführungsform der Erfindung weist die Vorrichtung eine unterbrochene Liegefläche auf.

Insbesondere ist der Bereich entlang der Mittellängsachse ausgespart. Hierdurch wird der Wirbelsäulenbereich der Person, die die Vorrichtung nutzt, in Bezug auf die Liegefläche ausgespart. Damit sind auch in diesem Bereich keine Elektroden angeordnet, was aus medizinischer Sicht sinnvoll ist. Die Aussparung entlang der Mittellängsachse ist darüber hinaus auch im Hinblick auf eine entspannte Lage der Benutzerperson zur Erhöhung des Stimulationseffektes sinnvoll. Darüber hinaus liegt die Person nicht mit ihren Dornfortsätzen auf der Vorrichtung auf, was unbequem bis schmerzhaft wäre. Außerdem ist hierdurch gewährleistet, dass die Elektroden an dem muskulären Teil des Rückens anliegen, wodurch eine Stimulation der Muskeln optimiert durchgeführt werden kann.

Die erfindungsgemäße Vorrichtung in Form einer Rückenauflage dient hauptsächlich zur Elektrostimulation des muskulären Rückens ohne die knöchernen Partien.

Die erfindungsgemäße Vorrichtung weist vorteilhaft mehrere Einzelauflagen auf, das heißt, die Liegefläche ist quer zu der Mittellängsachse in wenigstens zwei Einzelauflagen unterteilt. Die erfindungsgemäße Vorrichtung kann eine Ausnehmung im Bereich der Mittellängsachse und/oder Ausnehmungen quer zur Mittellängsachse aufweisen.

Hierdurch erhöht sich die Beweglichkeit der Silikonauflage, wodurch sich wiederum der Entspannungseffekt erhöht. Darüber hinaus liegen die Elektroden bei der Ausgestaltung mit Einzelauflagen besser am Körper der Person an, wodurch der Stimulationseffekt erhöht wird.

Vorteilhaft ist in jeder Einzelauflage jeweils wenigstens eine Elektrode angeordnet. Gemäß einer besonders bevorzugten Ausführungsform der Erfindung ist in jeder Einzelauflage jeweils eine Elektrode angeordnet. Dadurch dass die Auflageflächen vorteilhaft beweglich, beispielsweise durch die Ausbildung aus Silikon ausgebildet sind, können sich die Elektroden optimal der Körperkontur der auf der Vorrichtung liegenden Person anpassen, so dass eine möglichst vollflächige Auflage und hierdurch eine optimale Elektrostimulation erfolgt.

Gemäß einer besonders bevorzugten Ausführungsform der Erfindung sind die Elektroden jeweils an der in der Mittellängsachse angeordneten Ausnehmung angrenzend angeordnet. Hierdurch ist gewährleistet, dass die Stimulation des gesamten Körpers oder Körperbereiches erfolgt, und zwar im zentrumsnahen Bereich. Die Elektroden sind vorteilhaft beabstandet voneinander und/oder elektrisch isoliert voneinander angeordnet, damit die Elektrostimulation durch einen Stromfluss durch die Muskeln erfolgt.

Vorteilhaft weisen die Elektroden zumindest teilweise unterschiedliche Größen hinsichtlich ihrer Auflagefläche auf. Hierdurch ist eine optimale Stimulation in den verschiedenen Bereichen, beispielsweise des Rückens möglich. So sind die Elektroden im Bereich des Kreuzbeines größer hinsichtlich ihrer Auflagefläche ausgebildet als die Elektroden im Schulterbereich oder im Bereich der Brustwirbelsäule. Die Elektroden im Bereich der Halswirbelsäule weisen vorteilhaft eine geringe Breite, dafür eine größere Länge als die restlichen Elektroden auf. Die größte Breite weisen vorteilhaft die Elektroden im Lendenwirbelbereich auf.

Gemäß einer weiteren vorteilhaften Ausführungsform der Erfindung ist die Stimulationsintensität jeder Elektrode einzeln einstellbar. Es ist auch möglich, die Stimulationsintensität von Gruppen von Elektroden einzustellen. Hierdurch ist eine individuelle Anpassung der Elektrostimulation möglich, wodurch die Stimulation sehr effektiv wird.

Das erfindungsgemäße Verfahren zur Ansteuerung von mehreren Elektroden in einer Vorrichtung zur Elektrostimulation, bei der die Elektroden in oder auf der Vorrichtung zur Elektrostimulation, die eine Teilkörper- oder Ganzkörperauflage aufweist, angeordnet sind, zeichnet sich dadurch aus, dass die Elektroden nacheinander oder gleichzeitig ansteuerbar ausgebildet sind, und dass die Elektroden bezüglich einer Dauer und/oder einer Intensität der Stimulation einzeln oder paarweise angesteuert werden, und dass während des Abschwellens eines ersten Elektrodenpaares das nächste benachbarte Elektrodenpaar hinsichtlich der Intensität anschwillt.

Die Einzelansteuerung oder die Ansteuerung hintereinander weist den vorteil auf, dass individuelle Stimulationsprogramme für jeden Nutzer erstellt werden können.

Vorteilhaft werden die Elektroden oder Elektrodenpaare hinsichtlich der Intensität wellenförmig nacheinander angesteuert. Die wellenförmige Ansteuerung ist vorteilhaft für eine effektive Elektrostimulation.

Gemäß einer weiteren vorteilhaften Ausführungsform der Erfindung werden die Elektrodenpaare hinsichtlich der Intensität anschwellend und wieder abschwellend angesteuert. Hierdurch lässt sich am besten die wellenförmige Ansteuerung umsetzen.

Für eine optimale wellenförmige Ansteuerung ist vorgesehen, dass während des Abschwellens eines ersten Elektrodenpaares das nächste benachbarte Elektrodenpaar hinsichtlich der Intensität anschwillt. während des Abschwellens wird das nächste benachbarte Elektrodenpaar anschwellend angesteuert. Beim Abschwellen dieses Elektrodenpaares wird das nächste benachbarte Elektrodenpaar anschwellend angesteuert und so weiter- Es läuft damit eine Art Welle hinsichtlich der Intensität der Elektrostimulation durch die Vorrichtung.

Für eine weitere Verbesserung der Elektrostimulation ist vorteilhaft vorgesehen, dass die Elektrodenpaare in einer oder mehreren Wellen hinsichtlich der Intensität an- und abschwellend angesteuert werden.

Gemäß einer besonders bevorzugten Ausführungsform der Erfindung ist vorgesehen, dass eine Stimulation mit einem Strom mit einer Niederfrequenz von 0 bis 1.000 Hertz (Hz) oder einer Mittelfrequenz von ein bis zehn Kilohertz (kHz) durchgeführt wird. Dieser Bereich ist für eine Muskelstimulation besonders vorteilhaft.

Für eine optimierte Stimulation ist vorgesehen, dass die Vorrichtung erwärmt wird. Hierdurch entspannen sich die Muskeln und die Elektrostimulation ist besonders effektiv.

Die Elektroden können paarweise angesteuert werden, dass heißt, jeweils die Elektroden, die beidseitig von der Mittellängsachse angeordnet sind oder Elektrodenpaare, die lediglich rechts oder links von der Mittellängsachse angeordnet sind. Es ist jedoch auch möglich, sämtliche Elektroden einzeln anzusteuern.

Das erfindungsgemäße Verfahren kann mit einer erfindungsgemäßen Vorrichtung und umgekehrt durchgeführt werden.

Weitere Merkmale und Vorteile der Erfindung ergeben sich anhand der zugehörigen Zeichnung, in der ein Ausführungsbeispiel der erfindungsgemäßen Vorrichtung nur beispielhaft dargestellt ist. In der Zeichnung zeigen:
- Fig. 1: eine erfindungsgemäße Vorrichtung in Draufsicht;
- Fig. 2: eine erfindungsgemäße Vorrichtung in perspektivischer Ansicht;
- Fig. 3: eine erfindungsgemäße Vorrichtung in Seitenansicht.

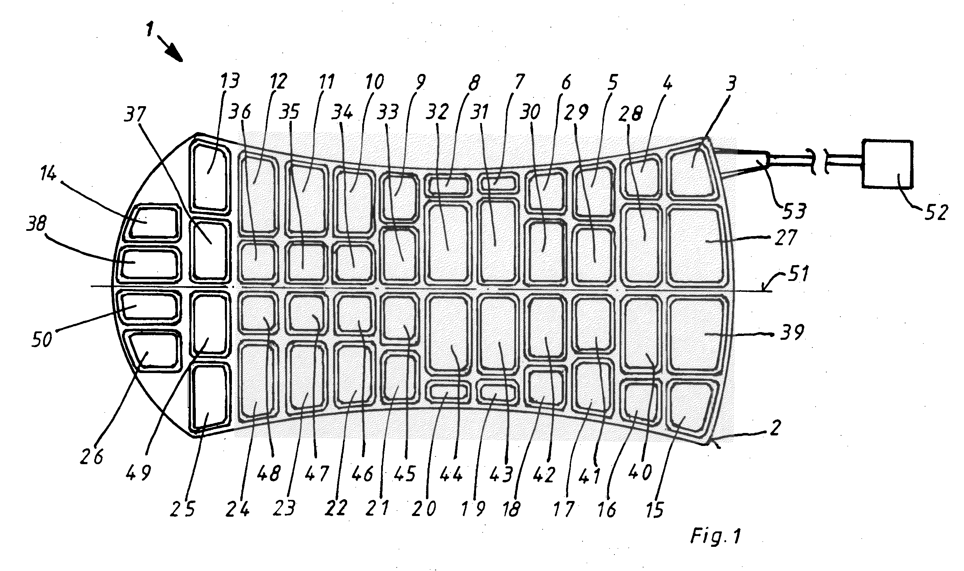

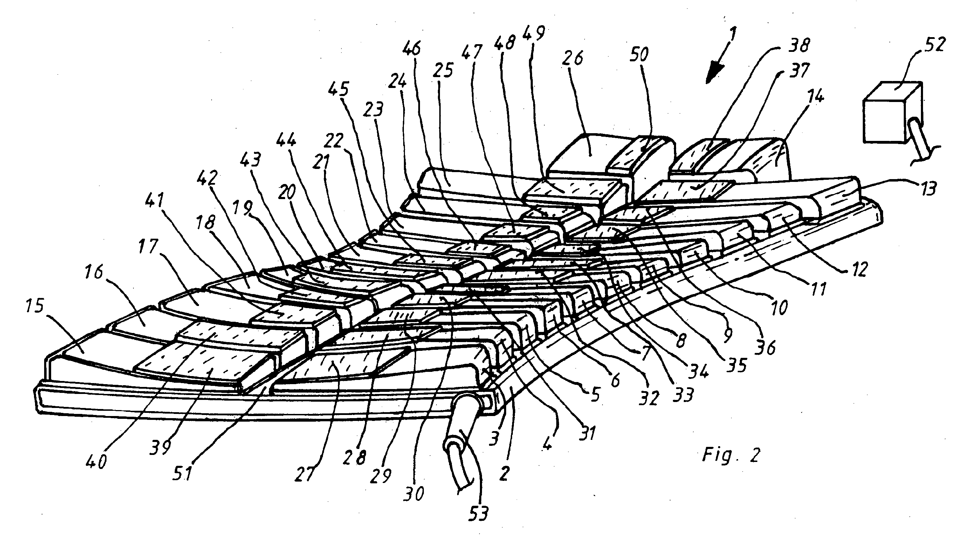

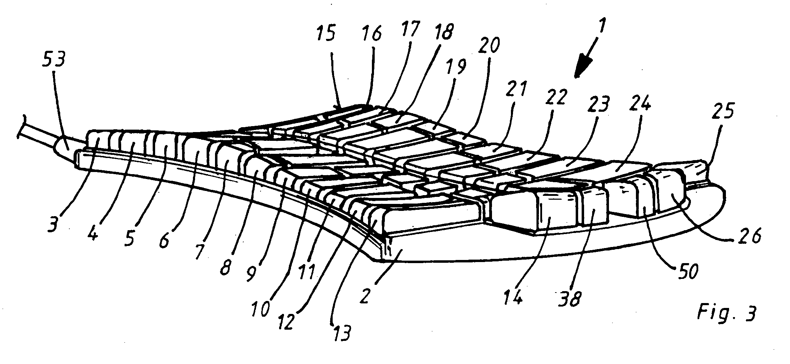

Die Fig. 1 bis 3 zeigen eine Vorrichtung 1, die einen Rahmen 2 und Einzelauflagen 3 bis 26 aufweist. In jeder Einzelauflage 3 bis 26 ist eine Elektrode 27 bis 50 angeordnet.

Die Vorrichtung 1 weist eine Mittellängsachse 51 auf. In der Mittellängsachse 51 liegend weist die Vorrichtung 1 eine Ausnehmung auf, das heißt hier ist keine Auflagefläche für Körperpartien vorgesehen.

Die Liegefläche, die aus den Einzelauflagen 3 bis 26 gebildet wird, ist einer Körperkontur angepasst ausgebildet.

Die Einzelauflagen 14, 26 dienen hierbei zur Auflage des Nacken-/Halswirbelbereiches und die Einzelauflagen 3, 15 dienen zur Auflage im Beckenbereich.

Die Einzelauflagen 3 bis 26 sind quer, das heißt im 90°-Winkel zur Mittellängsachse 51 mit dazwischen liegenden Ausnehmungen ausgebildet. Hierdurch ist es möglich, dass sich die gesamte Liegefläche einem Körper optimal anpasst, so dass die Elektroden 27 bis 50 eine maximale Anlagefläche an dem Körper (nicht dargestellt) aufweisen.

Die Elektroden 27, 39; 28, 40; 29, 41; 30, 42; 31, 43; 32, 44; 33, 45; 34, 46; 35, 47; 36, 48; 37, 49; 38, 50 bilden jeweils Paare, die auch paarweise von einer Steuervorrichtung 52 mit einer Steuerelektronik angesteuert werden können.

Es ist auch möglich, die Elektroden 39, 40, 41, 42, 43, 44, 45, 46, 47, 48, 49, 50 auf einer Seite der Vorrichtung 1 anzusteuern, beispielsweise in der beschriebenen Wellenform, ohne dass die Elektroden 27, 28, 29, 30, 31, 32, 33, 34, 35, 36, 37, 38 auf der anderen Seite der Mittellängsachse 51 angesteuert werden.

Die Ansteuerung der Elektrodenpaare 27, 39; 28, 40; 29, 41; 30, 42; 31, 43; 32, 44; 33, 45; 34, 46; 35, 47; 36, 48; 37, 49; 38, 50 kann folgendermaßen erfolgen:
Die Elektroden 27, 39 werden anschwellend und anschließend wieder abschwellend angesteuert. Während des Abschwellens werden die benachbarten Elektroden 28, 40 anschwellend angesteuert. Während die Elektroden 28, 40 wieder abschwellend angesteuert werden, werden die Elektroden 29, 41 anschwellend angesteuert und so weiter. Diese Vorgehensweise bildet eine Stimulationswelle in der Vorrichtung 1, mit der der Körper (nicht dargestellt) stimuliert wird.

Während der Ansteuerung der Elektroden 27, 39; 28, 40 werden keine weiteren Elektrodenpaare angesteuert, so dass eine einzige Welle die Vorrichtung 1 durchläuft.

Es ist jedoch auch möglich, dass beispielsweise, wenn die Stimulationswelle bis zu den Elektrodenpaaren 36, 48; 37, 49 durchgelaufen ist, die nächste Welle in den Elektrodenpaaren 27, 39; 28, 40 startet, so dass in diesem Fall zwei Stimulationswellen die Vorrichtung 1 durchlaufen.

Es ist auch möglich, dass noch mehr Wellen gleichzeitig die Vorrichtung 1 durchlaufen.

Die Ansteuerung erfolgt mittels der Steuervorrichtung 52, die lediglich schematisch dargestellt ist.

Die Einzelauflagen 3 bis 26 können erwärmt werden.

Vorteilhaft sind die Einzelauflagen 3 bis 26 sowie der Rahmen 2 aus Silikon gebildet.

Es ist jedoch auch möglich, andere Materialien vorzusehen.

Die Elektroden 27, 28, 39, 40 liegen im Kreuzbeinbereich. Die Elektroden 29, 41 sind im Kreuzbein- beziehungsweise im unteren Lendenwirbelbereich angeordnet. Der Lendenwirbelbereich erstreckt sich über die Elektroden 30, 31, 32, 42, 43, 44. Zum Brustwirbelbereich zählen die Elektroden 33, 34, 35, 36, 45, 46, 47, 48. Die Elektroden 37, 49 sind im Schulterbereich und beginnenden Halswirbelbereich angeordnet. Die Elektroden 38, 50 gehören zum Halswirbelbereich.

Die Elektroden 27, 28, 39, 40 im Kreuzbeinbereich sowie die Elektroden 31, 32, 43, 44 im Lendenwirbelbereich weisen die größte Breite auf, das heißt die größte Ausdehnung von der Mittellängsachse im 90°-Winkel ausgehend.

Die Elektroden 38, 50 im Halswirbelbereich weisen die geringste Breite auf. Dafür sind diese Elektroden 38, 50 länger als die übrigen Elektroden 27 bis 37, 39 bis 49 bezonen auf die Richtung der Mittellängsachse 51 ausgebildet. Die Elektroden 33, 34, 35, 36, 45, 46, 47, 48 im Brustwirbelbereich sind breiter als die Elektroden 38, 50 im Halswirbelsäulenbereich. Diese Elektroden 33, 34, 35, 36, 45, 46, 47, 48 im Brustwirbelbereich sind jedoch schmaler als die übrigen Elektroden außerhalb des Halswirbelbereiches.

Die Vorrichtung 1 weist einen Anschluss 53 für ein Kabel (abgebrochen dargestellt) auf, über das die Elektroden (27 bis 50) mit Strom versorgt und von der Steuervorrichtung 52 angesteuert werden.

### Bezugszahlen

- 1: Vorrichtung
- 2: Rahmen
- 3 bis 26: Einzelauflagen
- 27 bis 50: Elektroden
- 51: Mittellängsachse
- 52: Steuervorrichtung
- 53: Kabelanschluss

## Patentansprüche

1. Vorrichtung zur Elektrostimulation von Muskeln mit einer Teilkörper- oder Ganzkörperauflage und in der Auflage angeordneten Elektroden,
bei der die Elektroden (27 bis 50) paarweise zu einer Mittellängsachse (51) der Vorrichtung (1) angeordnet sind, wobei die Elektroden (27 bis 50) flächig ausgebildet sind, und wobei Elektrodenpaare (27 bis 50), die aus benachbarten Elektroden (27 bis 50) gebildet sind, nacheinander oder gleichzeitig ansteuerbar ausgebildet sind, wobei die Elektrodenpaare (27 bis 50) wellenförmig nacheinander ansteuerbar ausgebildet sind, **dadurch gekennzeichnet,**
- **dass** die Elektrodenpaare (27 bis 50) anschwellend und wieder abschwellend ansteuerbar ausgebildet sind,
- **dass** während des Abschwellens eines vorhergehenden Elektrodenpaares (27, 39) ein Anschwellen des nächsten benachbarten Elektrodenpaares (28, 40) vorgesehen ist,
- **dass** wenigstens ein Teil der Elektroden (27 bis 50) eine gekrümmte Oberfläche aufweist, und/oder
- **dass** wenigstens ein Teil der Elektroden (27 bis 50) zu einer Grundfläche der Auflage geneigt angeordnet ist, und
- **dass** die gekrümmte Oberfläche der Elektroden (27 bis 50) als eine an eine Körperkontur angepasste, gekrümmte Oberfläche ausgebildet ist und/oder
- **dass** die Neigung der Elektroden (27 bis 50) als eine an eine Körperkontur angepasste Neigung ausgebildet ist,
- **dass** die Vorrichtung (1) eine einer Körperkontur angepasste Liegefläche aufweist,
- **dass** die Liegefläche in einer Mittellängsachse (51) eine durchgehende Ausnehmung aufweist,
- **dass** die Vorrichtung (1) eine unterbrochene Liegefläche aufweist, und
- **dass** Auflageflächen beweglich ausgebildet sind, derart, dass die Elektroden sich optimal einer Körperkontur einer auf der Vorrichtung liegenden Person anpassend ausgebildet sind.

2. Vorrichtung nach Anspruch 1, **dadurch gekennzeichnet, dass** je zwei Elektroden (27 bis 50) paarweise zu der Mittellängsachse (51) der Vorrichtung (1) angeordnet sind.

3. Vorrichtung nach einem der vorhergehenden Ansprüche, **dadurch gekennzeichnet, dass** wenigstens sechs Elektrodenpaare (27 bis 50) vorgesehen sind.

4. Vorrichtung nach einem der vorhergehenden Ansprüche, **dadurch gekennzeichnet, dass** wenigstens zwölf Elektrodenpaare (27 bis 50) vorgesehen sind.

5. Vorrichtung nach einem der vorhergehenden Ansprüche, **dadurch gekennzeichnet, dass** die Elektrodenpaare (27 bis 50) in einer oder mehreren Wellen an- und abschwellend ansteuerbar ausgebildet sind.

6. Vorrichtung nach einem der vorhergehenden Ansprüche, **dadurch gekennzeichnet, dass** eine Stimulation mit einem Strom mit einer Niederfrequenz zwischen 0 und 1.000 Hertz (Hz) oder einer Mittelfrequenz zwischen ein und zehn Kilohertz (kHz) vorgesehen ist.

7. Vorrichtung nach einem der vorhergehenden Ansprüche, **dadurch gekennzeichnet, dass** die Vorrichtung (1) erwärmbar ausgebildet ist.

8. Vorrichtung nach einem der vorhergehenden Ansprüche, **dadurch gekennzeichnet, dass** ein Grundkörper der Vorrichtung (1) aus Silikon gebildet ist.

9. Vorrichtung nach Anspruch 10, **dadurch gekennzeichnet, dass** die Vorrichtung (1) wenigstens zwei Einzelauflagen (3 bis 26) aufweist.

10. Vorrichtung nach Anspruch 1 oder 9, **dadurch gekennzeichnet, dass** die Vorrichtung (1) in jeder Einzelauflage (3 bis 26) jeweils wenigstens eine Elektrode (27 bis 50) aufweist.

11. Vorrichtung nach Anspruch 1, **dadurch gekennzeichnet, dass** die Elektroden (27 bis 50) an der in der Mittellängsachse angeordneten Ausnehmung angrenzend angeordnet sind.

12. Vorrichtung nach einem der vorhergehenden Ansprüche, **dadurch gekennzeichnet, dass** die Elektroden (27 bis 50) zumindest teilweise unterschiedliche Größen hinsichtlich ihrer Auflagefläche aufweisen.

13. Vorrichtung nach einem der vorhergehenden Ansprüche, **dadurch gekennzeichnet, dass** die Elektroden (27 bis 50) einzeln ansteuerbar hinsichtlich der Stimulationsintensität ausgebildet sind.

14. Verfahren zur Ansteuerung von mehreren Elektroden in einer Vorrichtung nach Anspruch 1 zur Elektrostimulation von Muskeln, wobei die Elektroden in oder auf der Vorrichtung zur Elektrostimulation, die eine Teilkörper- oder Ganzkörperauflage aufweist, angeordnet sind,
**dadurch gekennzeichnet, dass** die Elektroden (27 bis 50) nacheinander oder gleichzeitig ansteuerbar ausgebildet sind, und dass die Elektroden (27 bis 50) bezüglich einer Dauer und/oder einer Intensität der Stimulation einzeln oder paarweise angesteuert werden, dass die Elektrodenpaare in einer oder mehreren Wellen hinsichtlich der Intensität an- und abschwellend angesteuert werden, und dass während des Abschwellens eines ersten Elektrodenpaares (27, 39) das nächste benachbarte Elektrodenpaar (28, 40) hinsichtlich der Intensität anschwillt.

15. Verfahren nach Anspruch 14, **dadurch gekennzeichnet, dass** die Elektroden (27 bis 50) oder Elektrodenpaare hinsichtlich der Intensität wellenförmig nacheinander angesteuert werden.

16. Verfahren nach Anspruch 14 oder 15, **dadurch gekennzeichnet, dass** die Elektroden (27 bis 50) oder Elektrodenpaare hinsichtlich der Intensität anschwellend und wieder abschwellend angesteuert werden.

17. Verfahren nach einem der Ansprüche 14 bis 16, **dadurch gekennzeichnet, dass** eine Stimulation mit einem Strom mit einer Niederfrequenz von 0 bis 1.000 Hertz (Hz) oder einer Mittelfrequenz von ein bis zehn Kilohertz (kHz) durchgeführt wird.

18. Verfahren nach einem der Ansprüche 14 bis 17, **dadurch gekennzeichnet, dass** die Vorrichtung (1) erwärmt wird.

19. Verfahren nach einem der Ansprüche 14 bis 18, **dadurch gekennzeichnet, dass** das Verfahren mit einer Vorrichtung gemäß einem oder mehreren der Ansprüche 1 bis 13 durchgeführt wird.

## Claims

1. Apparatus for electrostimulation of muscles, having a support for part of the body or for the whole body, and electrodes disposed in the support,
wherein the electrodes (27 to 50) are disposed in pairs relative to a centre longitudinal axis (51) of the apparatus (1), wherein the electrodes (27 to 50) are configured to be planar, and wherein electrode pairs (27 to 50) that are formed from adjacent electrodes (27 to 50) are configured so that they can be controlled one after the other or simultaneously, wherein the electrode pairs (27 to 50) are configured so that they can be controlled in wave form, one after the other, **characterised**
- **in that** the electrode pairs (27 to 50) are configured so that they can be controlled to increase and decrease again,
- **in that** during the decrease of a preceding electrode pair (27, 39), an increase of the next, adjacent electrode pair (28, 40) is provided,
- **in that** at least a part of the electrodes (27 to 50) has a curved surface, and/or
- **in that** at least a part of the electrodes (27 to 50) is disposed inclined relative to a base surface of the support, and
- **in that** the curved surface of the electrodes (27 to 50) is configured as a curved surface adapted to a body contour and/or
- **in that** the inclination of the electrodes (27 to 50) is configured as an inclination adapted to a body contour,
- **in that** the apparatus (1) has a lying surface adapted to a body contour,
- **in that** the lying surface has a continuous recess in the centre longitudinal axis (51),
- **in that** the apparatus (1) has an interrupted lying surface, and
- **in that** contact surfaces are configured to be movable, such that the electrodes are configured to optimally adapt to a body contour of a person lying on the apparatus.

2. Apparatus according to claim 1, **characterised in that** two electrodes (27 to 50), in each instance, are disposed in pairs relative to the centre longitudinal axis (51) of the apparatus (1).

3. Apparatus according to one of the preceding claims, **characterised in that** at least six electrode pairs (27 to 50) are provided.

4. Apparatus according to one of the preceding claims, **characterised in that** at least twelve electrode pairs (27 to 50) are provided.

5. Apparatus according to one of the preceding claims, **characterised in that** the electrode pairs (27 to 50) are configured so that they can be controlled to increase and decrease, in one or more waves.

6. Apparatus according to one of the preceding claims, **characterised in that** stimulation is provided with a current having a low frequency between 0 and 1,000 hertz (Hz) or a medium frequency between one and ten kilohertz (kHz).

7. Apparatus according to one of the preceding claims, **characterised in that** the apparatus (1) is configured so that it can be heated.

8. Apparatus according to one of the preceding claims, **characterised in that** a base body of the apparatus (1) is formed from silicone.

9. Apparatus according to claim 10, **characterised in that** the apparatus (1) has at least two individual supports (3 to 26).

10. Apparatus according to claim 1 or 9, **characterised in that** the apparatus (1) has at least one electrode (27 to 50), in each instance, in each individual support (3 to 26).

11. Apparatus according to claim 1, **characterised in that** the electrodes (27 to 50) are disposed bordering on the recess disposed in the centre longitudinal axis.

12. Apparatus according to one of the preceding claims, **characterised in that** the electrodes (27 to 50) have different sizes, at least in part, with regard to their contact surface.

13. Apparatus according to one of the preceding claims, **characterised in that** the electrodes (27 to 50) are configured so that they can be controlled individually with regard to the stimulation intensity.

14. Method for controlling a plurality of electrodes in an apparatus according to claim 1 for electrostimulation of muscles, wherein the electrodes are disposed in or on the apparatus for electrostimulation, which has a support for part of the body or for the whole body,
**characterised in that** the electrodes (27 to 50) are configured so that they can be controlled one after the other or simultaneously, and **in that** the electrodes (27 to 50) are controlled individually or in pairs, with regard to a duration and/or an intensity of the stimulation, **in that** the electrode pairs are controlled to increase and decrease in one or more waves, with regard to intensity, and **in that** while a first electrode pair (27, 39) is decreasing, the next, adjacent electrode pair (28, 40) is increasing, with regard to intensity.

15. Method according to claim 14, **characterised in that** the electrodes (27 to 50) or electrode pairs are controlled in wave form, one after the other, with regard to intensity.

16. Method according to one of claims 14 or 15, **characterised in that** the electrodes (27 to 50) or electrode pairs are controlled to increase and decrease again, with regard to intensity.

17. Method according to one of claims 14 to 16, **characterised in that** stimulation is carried out with a current having a low frequency from 0 to 1,000 hertz (Hz) or a medium frequency from one to ten kilohertz (kHz).

18. Method according to one of claims 14 to 17, **characterised in that** the apparatus (1) is heated.

19. Method according to one of claims 14 to 18, **characterised in that** the method is carried out with an apparatus according to one or more of claims 1 to 13.

## Revendications

1. Dispositif pour l'électro-stimulation de muscles avec un support corporel partiel ou entier et des électrodes disposées dans le support,
dans lequel les électrodes (27 à 50) sont disposées par paires par rapport à un axe longitudinal central (51) du dispositif (1), les électrodes (27 à 50) présentant une forme plane et les paires d'électrodes (27 à 50), qui constituées d'électrodes adjacentes (27 à 50), sont conçues de façon à pouvoir être pilotées successivement ou simultanément,
les paires d'électrodes (27 à 50) étant conçues de façon à pouvoir être pilotées successivement à la manière d'une onde,
**caractérisé en ce que**
- les paires d'électrodes (27 à 50) sont conçues de façon à pouvoir être pilotées avec un gonflement suivi d'un dégonflement,
- pendant le dégonflement d'une paire d'électrodes précédente (27, 39), un gonflement de la paire d'électrodes suivante (28,40) est prévu,
- au moins une partie des électrodes (27 à 50) présente une surface incurvée, et/ou
- au moins une partie des électrodes (27 à 50) est disposée de manière inclinée par rapport à une surface de base du support, et
- la surface incurvée des électrodes (27 à 50) est conçue comme une surface incurvée adaptée à un contour du corps, et/ou
- l'inclinaisan des électrodes (27 à 50) est conçue comme une inclinaison adaptée à un contour du corps,
- le dispositif (1) comprend une surface de couchage adapté à un contour du corps,
- la surface de couchage comprend un évidement traversant dans un axe longitudinal central (51),
- le dispositif (1) comprend une surface de couchage interrompue, et
- des surfaces de support sont conçues de manière mobile, de façon à ce que les électrodes sont conçues afin de s'adapter de manière optimale au contour du corps d'une personne couchée sur le dispositif.

2. Dispositif selon la revendication 1, **caractérisé en ce que** deux électrodes (27 à 50) sont disposées par paires par rapport à l'axe longitudinal central (51) du dispositif (1).

3. Dispositif selon l'une des revendications précédentes, **caractérisé en ce qu'**au moins six paires d'électrodes (27 à 50) sont prévues.

4. Dispositif selon l'une des revendications précédentes, **caractérisé en ce qu'**au moins douze paires d'électrodes (27 à 50) sont prévues.

5. Dispositif selon l'une des revendications précédentes, **caractérisé en ce que** les paires d'électrodes (27 à 50) sont conçues de façon à pouvoir être pilotées avec un gonflement et un dégonflement en une ou plusieurs vagues.

6. Dispositif selon l'une des revendications précédentes, **caractérisé en ce qu'**une stimulation est prévue avec une basse fréquence entre 0 et 1000 Hertz (Hz) ou une fréquence moyenne entre un et dix kilohertz (kHz).

7. Dispositif selon l'une des revendications précédentes, **caractérisé en ce que** le dispositif (1) est conçu de façon à pouvoir être chauffé.

8. Dispositif selon l'une des revendications précédentes, **caractérisé en ce qu'**un corps de base du dispositif (1) est constitué de silicone.

9. Dispositif selon la revendication 10, **caractérisé en ce que** le dispositif (1) comprend au moins deux supports individuels (3 à 26).

10. Dispositif selon la revendication 1 ou 9, **caractérisé en ce que** le dispositif (1) comprend, dans chaque support individuel (3 à 26), au moins une électrode (27 à 50).

11. Dispositif selon la revendication 1, **caractérisé en ce que** les électrodes (27 à 50) sont disposées à proximité de l'évidement se trouvant dans l'axe longitudinal central.

12. Dispositif selon l'une des revendications précédentes, **caractérisé en ce que** les électrodes (27 à 50) présentent, en ce qui concerne leur surface d'appui, des tailles au moins partiellement différentes.

13. Dispositif selon l'une des revendications précédentes, **caractérisé en ce que** les électrodes (27 à 50) sont conçues de façon à pouvoir être pilotées individuellement en ce qui concerne l'intensité de la stimulation.

14. Procédé de pilotage de plusieurs électrodes dans un dispositif selon la revendication 1 pour l'électro-stimulation de muscles, les électrodes étant disposées dans ou sur le dispositif d'électro-stimulation, qui comprend un support corporel partiel ou entier, **caractérisé en ce que** les électrodes (27 à 50) sont conçues de façon à pouvoir être pilotées successivement ou simultanément et **en ce que** les électrodes (27 à 50) sont pilotées, en ce qui concerne une durée et/ou une intensité de stimulation, de manière individuelle ou par paires, **en ce que** les paires d'électrodes sont pilotées en une ou plusieurs vagues en ce qui concerne l'intensité, avec un gonflement et un dégonflement et **en ce que**, pendant le dégonflement d'une première paire d'électrodes (27, 39), la paire d'électrode adjacente suivante (28, 40) gonfle en ce qui concerne l'intensité.

15. Procédé selon la revendication 14, **caractérisé en ce que** les électrodes (27 à 50) ou les paires d'électrodes sont pilotées successivement soues la forme d'une vague en ce qui concerne l'intensité.

16. Procédé selon la revendication 14 ou 15, **caractérisé en ce que** les électrodes (27 à 50) ou les paires d'électrodes sont pilotées, en ce qui concerne l'intensité, avec un gonflement puis un dégonflement.

17. Procédé selon l'une des revendications 14 à 16, **caractérisé en ce qu'**une stimulation est effectuée avec un courant avec une basse fréquence de 0 à 1000 Hertz (Hz) ou une fréquence moyenne de un à dix kilohertz (kHz).

18. Procédé selon l'une des revendications 14 à 17, **caractérisé en ce que** le dispositif (1) est chauffé.

19. Procédé selon l'une des revendications 14 à 18, **caractérisé en ce que** le procédé est effectué avec un dispositif selon l'une ou plusieurs des revendications 1 à 13.
